# EUROPÄISCHE PATENTSCHRIFT

(11) **EP 1 386 158 B1**
(45) Veröffentlichungstag und Bekanntmachung des Hinweises auf die Patenterteilung: **14.05.2008**
(21) Anmeldenummer: 01940432.6
(22) Anmeldetag: 09.05.2001
(51) Int. Cl.: G01N 33/543

(54) **GEGENSTAND MIT EINER UNGELADENEN, FUNKTIONALISIERTEN HYDROGELOBERFLÄCHE**
OBJECT COMPRISING AN UNCHARGED, FUNCTIONALIZED HYDROGEL SURFACE
OBJET A SURFACE HYDROGEL FONCTIONNALISEE NON CHARGEE

(43) Veröffentlichungstag der Anmeldung: 04.02.2004
(73) Patentinhaber: Hofmann, Andreas, 96346 Wallenfels (DE)
(72) Erfinder: Hofmann, Andreas, 96346 Wallenfels (DE)
(74) Vertreter: Sandmann, Joachim
(86) Internationale Anmeldenummer: PCT/EP2001/005288
(87) Internationale Veröffentlichungsnummer: WO 2002/090988

(56) Entgegenhaltungen:
- WO-A-92/03732
- WO-A-97/41425
- DE-A- 19 817 180
- DE-A- 19 916 638
- US-A- 5 242 828

## Beschreibung

Die Erfindung betrifft einen Gegenstand mit einer ungeladenen, funktionalisierten Oberfläche umfassend ein Hydrogel, das Hydroxygruppen aufweist, an das einen oder mehrere Reste A sowie einen oder mehrere Reste B aufweisende organische Moleküle über die Reste A gebunden sind, wobei die Reste B mit Aminogruppen oder Thiogruppen reagieren können und wobei die Hydroxygruppen selektiv mit dem Rest A bzw. den Resten A reagiert haben.

Bekannt aus DE 199 16 638 A ist ein Gegenstand mit einer funktionalisierten Oberfläche umfassend ein Hydrogel, an das über Reste A organische Moleküle gebunden sind, wobei die eingesetzten organischen Moleküle einen oder mehrere Reste A, die mit Hydroxygruppen reagieren können, und einen oder mehrere Reste B aufweisen, die mit Aminogruppen oder Thiogruppen reagieren können. Dabei handelt es sich jedoch nicht um eine ungeladene funktionalisierte Oberfläche mit Hydroxygruppen.

Vielmehr ist Stand der Technik die Immobilisierung von Rezeptoren an geladenen Hydrogelen, z.B. Carboxymethyldextran COO- oder Aminodextran NH2+ (EP 589 867 B). Das eingesetzte Hydroxypolymer wird vor Anbringung der Rezeptormoleküle - vorwiegend mit Bromessigsäure - in ein Carboxymethylpolymer und somit in ein geladenes Hydrogel umgewandelt.

Oberflächen für biotechnologische Anwendungen können mit kurzen Linkermolekülen funktionalisiert sein, die auf Grund ihrer chemischen Reaktivität die Anbindung von Biomolekülen gestatten, ohne dass zusätzliche Aktivierungsreagenzien benötigt werden (Katalog Pierce Coated Microwell Plates, 1998). Diese Oberflächen neigen Jedoch zu unspezifischen Adsorptionen, die zu einer Verfälschung des Meßsignals führen. Bei den Meßverfahren, die auf Affinitätswechselwirkung basieren, z. B. bei der biochemischen Interaktionsanalyse mit Hilfe der Oberflächenplasmonenresonanz (SPR), wird in diesem Fall eine höhere Konzentration von Biomolekülen vorgetäuscht als tatsächlich In der Lösung vorhanden Ist.

Es Ist ferner bekannt, Oberflächen mit Hydrogelschichten zu versehen, um unspezifische Adsorpdcnsptlänomene zu unterdrücken, wobei das Hydrogel zusätzliche funktionelle Reste aufweist, die die Anbindung von Biomolekülen ermöglichen. Als funktionelle Reste werden nach dem Stand der Technik Gruppen, wie z. B. Carboxy- oder Aminogruppen, eingesetzt, die In Abhängigkeit vom pH-Wert Ladungen aufweisen und die in einem weiteren Schritt mit einem Aktivierungsreagenz umgesetzt werden müssen, bevor ein interessierendes Molekül gebunden werden kann (Sensing Surfaces Capable of Selective Biomolecular Interactions, To Be Used In Sensor Systems, EP-B-0589867). Dies hat zwei Nachteile zur Folge: zum einen besteht die Möglichkeit, daß durch unvollständige Umsetzung geladene Gruppen an der Oberfläche vorhanden bleiben, so daß Ober elektrostatische Wechselwirkungen unspezifische Adsorption erfolgen kann, zum anderen ist die Verwendung eines Aktivierungsreagenz für den Endanwender ein zusätzlicher Arbeitsschritt, der auch zusätzliche Fehlermöglichkeiten birgt.

Ziel der Erfindung ist es daher, Gegenstände, wie Sensoren, Quarzwaagen oder Gegenstände, die Mikrokavitäten aufweisen, mit Oberflächen bereitzustellen, die die Nachteile der bekannten Systeme vermeiden und für den Endanwender leicht handhabbar sind. Eine weitere Aufgabe der Erfindung ist es, ein Verfahren zur Herstellung eines Gegenstandes der genannten Art bereitzustellen. Weiterhin sollen neue Verbindungen bereitgestellt werden.

Gegenstand der Erfindung ist eine ungeladene funktionalisierte Oberfläche gemäß Anspruch 1.

Bei einem bevorzugten erfindungsgemäßen Gegenstand sind die eingesetzten organischen Moleküle ein oder mehrere Reste A ausgewählt aus Säurechloridgruppen und Diazogruppen und ein oder mehrere Reste B ausgewählt aus Vinylsulfongruppen, N-Hydroxysuccinimidestergruppen und Maleimidgruppen.

Weiterhin betrifft die Erfindung ein Verfahren zur Herstellung eines Gegenstandes mit einer ungeladenen, funktionalisierten Oberfläche, umfassend die Schritte:
(a) Bereitstellen eines Gegenstandes mit einer nicht funktionalisierten Hydrogeloberfläche, wobei das Hydrogel Hydroxygruppen aufweist:
(b) Kovalentes Anbinden an das Hydrogel von organischen Molekülen, die einen oder mehrere Reste A, die mit Hydroxygruppen reagieren können, und einen oder mehrere Reste B, die mit Aminogruppen oder Thiogruppen reagieren können, aufweisen,
wobei die organischen Moleküle selektiv über den Rest A bzw. die Reste A mit den Hydroxygruppen des Hydrogels reagieren.

Erfindungsgemäß wird der oben beschriebene Gegenstand zur Anbindung von Biomolekülen mit Amino- oder Thiogrupper verwendet. Diese Biomoleküle dienen als Rezeptoren für Analytmoleküle.

Die erfindungsgemäßen Gegenstände können als Mikrokavitäten oder in den verschiedensten analytischen Meßverfahren, wie z. B. Oberflächenplasmonenresonanz (SPR), Quarzwaagen, oder interferometrischen Meßmethoden, z. B. Reflektionsinterferenzkontrastmikroskopie, eingesetzt werden. Besonders eignen sie sich zum Einsatz in der SPR. Der Aufbau der nicht funktionalisierten Oberfläche des erfindungsgemäßen Gegenstandes richtet sich nach dem analytischen Verfahren, in dem der erfindungsgemäße Gegenstand eingesetzt werden soll, und ist dem Fachmann bekannt (Joumal of Biomedical Materials Research, 18 (953-959) (1984) und J. Chem. Soc., Chem. Commun., 1990, 1526).

Im Rahmen der Erfindung wird der Begriff "nicht funktionalisierte Oberfläche" verwendet, um die Oberfläche eines Gegenstandes mit der Hydrogelschicht vor der Anbindung der organischen Moleküle mit den Resten A und B zu bezeichnen. Der Begriff "funktionalisierte Oberfläche" wird verwendet, um die Oberfläche eines Gegenstandes - mit der Hydrogelschicht nach der Anbindung der organischen Moleküle mit den Resten A und B zu bezeichnen. Im Rahmen der Erfindung bedeutet "ungeladen", daß im pH-Bereich zwischen 4 und 11, bevorzugt zwischen 5 und 9, weniger als 0,1% aller funktionellen Gruppen am Hydrogel in einem geladenen Zustand vorliegen. Den Ladungszustand dieser Gruppen kann man über ihren pKₐ-Wert berechnen.

Der erfindungsgemäße Gegenstand besitzt eine Grundoberfläche umfassend z. B. eine Glas-, Metall- oder Kunststoffschicht. Bevorzugte Metallschichten sind Edelmetallschichten z. B. aus Gold oder Silber wie sie z. B. in der SPR Verwendung finden. Als Kunststoffschichten sind in den entsprechenden Anwendungen z. B. bei Mikrokavitäten, beispielsweise Polyethylen, Polypropylen, Polystyrol und Makrolon^{®} bekannt.

Gemäß der vorliegenden Erfindung ist es wesentlich, daß der nicht funktionalisierte Gegenstand eine Hydrogelschicht an der Oberfläche aufweist. Diese Schicht dient zur Verhinderung unspezifischer Adsorptionen, die das Meßsignal verfälschen. Hydrogele sind mit Wasser quellbare Polymere. Um die Anbindung der organischen Moleküle mit den Resten A zu ermöglichen, müssen die Hydrogele Hydroxygruppen aufweisen. Bei den Hydrogelen kann es sich z. B. um ein Polysaccharid, ein Derivat davon oder um ein quellbares organisches Polymer wie Poly{N-[tris-(hydroxymethyl)-methyl]-acrylsäureamid}, Polyvinylalkohol oder Polyethylenglykol mit endständigen Hydroxygruppen handeln. Bevorzugt sind Polysaccharide. Beispiele für Polysaccharide sind Amylose, Inulin, Pullulan oder Dextran. Bevorzugt sind Pullulan oder Dextran. Besonders bevorzugt ist Dextran.

Die Hydrogelschicht sollte mehrere Nanometer dick sein. Sie quillt im wäßrigen Milieu zu einer Dicke von ca. 100 nm auf, wodurch die Oberfläche vollständig bedeckt wird. Die gequollene Polymerschicht ahmt die natürliche Umgebung von Biomolekülen nach und ist geeignet, eine Denaturierung und somit Inaktivierung der Biomolekülen zu verhindern. Zusätzlich wird die Adsorption von anderen als den zu analysierenden Molekülen wirksam unterdrückt. Außerdem ist die gequollene Hydrogelschicht in der Lage, Unregelmäßigkeiten der Oberfläche auszugleichen: Die Anbindung der Linkermoleküle und damit auch der Biomoleküle findet auch in der gequollenen Matrix und nicht nur unmittelbar an der Oberfläche statt. Hierdurch verringert sich die Bedeutung von Oberflächenunebenheiten, die sonst zu einer schlecht definierten Oberfläche und dadurch zu schlecht quantifizierbaren Meßergebnissen beitragen.

Verfahren zur Beschichtung von Oberflächen mit Hydrogelen sind bekannt und variieren in Abhängigkeit vom gewählten Gegenstand, z. B. Sensor oder Mikrokavität (J. of Biomedical Materials Research, 18, 953 (1984), DE-A 198 17 180). Beispielsweise wird eine entsprechend vorbereitete Oberfläche (Anmeldung DE-A 198 17 180, EP-B-0 589 867) zwischen 1 Stunde und 5 Stunden, typischerweise 3 Stunden, in eine entsprechende, frisch bereitete wäßrige Hydrogellösung, hergestellt aus Hydroxypolymer, gegeben. Die Konzentration des Hydroxypolymers in der Lösung liegt zwischen 10 und 500 mg•ml⁻¹.

An diese nicht funktionalisierte Hydrogelschicht werden difunktionelle organische Moleküle gebunden, die mindestens einen Rest A und mindestens einen Rest B aufweisen. Die Reste A und B werden so gewählt, daß bei der Anbindung des organischen Moleküls an das Hydrogel selektiv der Rest A mit dem Hydrogel reagiert. Da die Selektivität der Anbindung an ein Hydrogel sich nur schwer quantifizieren läßt wird im Rahmen dieser Erfindung der Grad der Selektivität über einen Modellversuch in Lösung ermittelt, wobei ein Alkohol als Modellverbindung für die Hydroxygruppen eines Hydrogels eingesetzt wird: 0,4 mol der zu untersuchenden organischen Verbindung werden mit 0,4 mol Isopropanol in trockenes Dichlormethan gegeben. Die Lösung wird 12 Stunden bei 25 °C gerührt. Nach Verdampfen des Lösungsmittels bei Unterdruck wird der Rückstand mit 500 ml Dichlormethan extrahiert. Die organische Phase wird mit 500 ml Wasser und 500 ml 0,1N Natriumhydroxidlösung gewaschen, über Magnesiumsulfat getrocknet und durch Verdampfen des Lösungsmittels konzentriert. Durch ¹H-NMR-Spektroskopie wird der jeweilige Anteil der Produkte bestimmt, die durch Reaktion des Restes A mit dem Alkohol entstanden sind, und die durch Reaktion des Restes B mit dem Alkohol entstanden sind. Dies wird durch Vergleich der Fläche unter geeignet ausgewählten Signalen der Produkte bestimmt. Im Rahmen der Erfindung bedeutet "selektiv", daß weniger als 5 % der organischen Moleküle über den Rest B mit dem Alkohol verbunden sind, bevorzugt sind weniger als 1 % der organischen Moleküle über den Rest B mit dem Alkohol verbunden. Unter "Anbindung" wird eine kovalente Reaktion zwischen dem Rest und dem Hydrogel verstanden.

Beispiele für den Rest A sind: Säurechloridgruppen -COCl und Diazogruppen 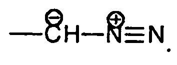 . Der Rest A reagiert mit den Hydroxygruppen des Hydrogels. Die Funktionalisierung der Hydrogelschicht mit den difunktionellen organischen Molekülen muß so erfolgen, daß die Oberfläche des Gegenstandes ungeladen ist. Die Menge der mit den difunktionellen organischen Molekülen umgesetzten Hydroxygruppen des Hydrogels beträgt bevorzugt zwischen 5 und 30%, besonders bevorzugt zwischen 8 und 15%.

Neben dem Rest A weisen die organischen Moleküle einen weiteren Rest B auf. Dieser Rest wird so gewählt, daß er einerseits unter den gewählten Reaktionsbedingungen bei der Anbindung der difunktionellen organischen Moleküle nicht reagiert und er andererseits, nach der Anbindung der difunktionellen organischen Moleküle an das Hydrogel, ohne Verwendung eines oder mehrerer Aktivierungsreagenzien mit einem Biomolekül mit Amino- oder Thiogruppen reagieren kann. Der Rest B soll so gewählt werden, daß er sofort ohne weitere Zwischenschritte mit einem Biomolekül mit Amino- oder Thiogruppen reagieren kann. Durch Auswahl der Reste A und B unter Beachtung der unterschiedlichen Reaktivität zu dem Hydrogel und zu dem Biomolekül mit Amino- oder Thiogruppen ist es somit möglich, ohne die Verwendung von Schutzgruppen für der Rest B zu arbeiten. Dies vereinfacht das Herstellungsverfahren des erfindungsgemäßen Gegenstandes und erspart zudem Kosten.

Als Rest B sind z. B. Vinylsulfongruppen (I), N-Hydroxysuccinimidestergruppen (II), Maleimidgruppen (III), oder andere Aktivestergruppen geeignet. * gibt die Bindungsstelle zu dem restlichen organischen Molekül an. Besonders bevorzugt sind Vinylsulfongruppen, N-Hydroxysuccinimidestergruppen und Maleimidgruppen.

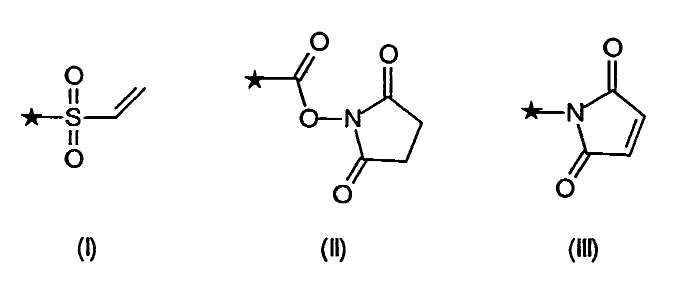

Die Reste A und B in den difunktionellen organischen Molekülen können durch einen Rest X verbunden sein. Die Wahl des Restes X kann in weiten Bereichen variieren, wobei er weder mit dem Hydrogel noch mit dem Biomolekül mit Amino- oder Thiogruppen reagieren soll, noch geladen sein soll. In den difunktionellen organischen Moleküle der Formel A-X-B ist der Rest X bevorzugt eine Einfachbindung oder eine verzweigte oder unverzweigte Kohlenwasserstoffkette, die eine Kettenlänge von bis zu 15 Kohlenstoffatomen besitzt und die bis zu zweimal jeweils durch eine Phenylengruppe oder ein heteroatomenthaltende Gruppe unterbrochen sein kann. Heteroatomenthaltende Gruppen sind z. B. -O-, -S-, -CONH- oder -COO-. Bei der Berechnung der Kettenlänge werden die Atome der heteroatomenthaltenden Gruppen bzw. die Kohlenstoffe der Phenylengruppen nicht mitgezählt. Die Kohlenwasserstoffkette weist bevorzugt eine Kettenlänge von bis zu 6 Kohlenstoffatomen auf. Die Kohlenwasserstoffkette ist vorzugsweise unverzweigt und weist vorzugsweise keine Phenylengruppen oder heteroatomenthaltende Reste auf. Die Bedingungen für die Synthese geeigneter Moleküle sind stark vom Einzelfall abhängig. Synthesewege für ausgewählte organische Moleküle sind in den Beispielen angegeben.

Beispiele für organische Moleküle der Formel A-X-B sind:

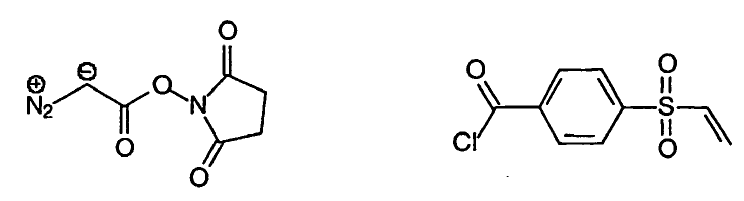

In einer weiteren möglichen Ausführungsform sind die Reste A und B an ein Polymer bzw. Oligomer gebunden. Das Polymer bzw. Oligomer muß mindestens einen Rest A und mindestens einen Rest B aufweisen. Bevorzugt weist das Polymer bzw. Oligomer an mindestens jeder fünften Wiederholungseinheit Reste A und/oder B auf. Die Reste A und B können jeweils unabhängig voneinander entweder über einen Spacer (d. h. eine Kohlenwasserstoffkette, die ggf. durch heteroatomenthaltende Einheiten, wie Amid, Ether, Sulfid, unterbrochen sein kann) oder direkt an das Rückgrat des Polymers bzw. Oligomers gebunden sein. Die Reste können entweder terminal oder nicht terminal an das Polymer gebunden sein.

Das Polymer bzw. Oligomer kann aus Monomeren hergestellt werden, die sowohl einen Rest A als auch einen Rest B aufweisen. Es ist aber ebenfalls möglich, das Polymer bzw. Oligomer aus Monomeren zu synthetisieren, wobei mindestens ein Monomer einen Rest A aufweist, während mindestens ein zweites Monomer einen Rest B aufweist. Es ist weiterhin möglich, ein Polymer bzw. Oligomer zu synthetisieren und es anschließend mit den Resten A und B zu derivatisieren. Polymerisationsverfahren sind dem Fachmann bekannt (Bruno Vollmert, Grundriß der Makromolekularen Chemie, Band 1, E. Vollmert-Verlag, Karlsruhe 1988).

Das Rückgrat des Polymers oder Oligomers kann in weiten Bereichen variieren, wobei es chemisch inert sein sollte, d. h. es sollte weder mit dem Hydrogel noch mit dem Biomolekül mit Amino- oder Thiogruppen reagieren, noch sollte er mit den Gruppen A und B reagieren. Weiterhin sollte es ungeladen sein. Geeignet sind z. B. Polyacrylsäureester, Polymethacrylsäureester, Polyacrylamide, Polyvinylverbindungen, Polystyrolderivate oder Copolymere hiervon. Besonders geeignet sind Polyacrylsäureester oder Polyacrylamide. Geeignete Polymere sollten eine Molmasse zwischen 5000 und 20000 aufweisen und in aprotischen organischen Lösungsmitteln löslich sein.

Die Verwendung niedermolekularer Verbindungen als Linker hat den Vorteil, daß man mit einer einheitlichen, definierten Verbindung arbeiten kann, die vor ihrer Verwendung auch leicht analysiert werden kann. Polymere oder Oligomere hingegen bieten den Vorteil, mehrere reaktive Gruppen pro Molekül tragen zu können. Dies erleichtert die kovalente Anbindung an die Hydrogeloberfläche.

Die Reaktionsbedingungen zur Kopplung der difunktionellen, organischen Moleküle an die Hydrogelschicht variieren in Abhängigkeit von den gewählten Resten A und B und in Abhängigkeit davon, ob niedermolekulare Verbindungen vom Typ A-X-B oder polymere bzw. oligomere Verbindungen eingesetzt werden. Beispiele für diese Reaktionsbedingungen werden nachstehend beschrieben.

Der erfindungsgemäße Gegenstand weist eine ungeladene Oberfläche auf. Zur Anbindung von Biomolekülen an Gegenständen, wie Sensoroberflächen, werden im Stand der Technik, z. T. in einem getrennten Verfahrensschritt, Aktivierungsreagenzien, wie z. B. Ethyl-(3-dimethylaminopropyl)-carbodiimid (EDC) und N-Hydroxysuccinimid (NHS), benötigt. Diese Aktivierungsreagenzien müssen an funktionelle Gruppen der Oberfläche gebunden werden, um diese in eine reaktive Form zu überführen, wodurch dann erst eine kovalente Bindung von Biomolekülen an die Oberfläche möglich wird. Da dieser Schritt von dem Endanwender durchgeführt werden muß, ist ein großes Interesse vorhanden an Gegenständen, wie Sensoren, die diese Zwischenbehandlung nicht benötigen. Durch die Verwendung von Aktivierungsreagenzien können die funktionellen Gruppen am Hydrogel nicht quantitativ umgesetzt werden, so daß eine kovalente Bindung von Biomolekülen mit Amino- oder Thiogruppen nur an einem Teil der dafür vorgesehenen funktionellen Gruppen erfolgt. Bisher war es nicht möglich, dem Anwender gebrauchsfertige Gegenstände mit einer ungeladenen, funktionalisierten Oberfläche, die eine Hydrogelschicht umfassen, zur Verfügung zu stellen, die in einem Schritt, ohne die Verwendung von Schutzgruppen oder Aktivierungsreagenzien, hergestellt wurden. Es waren keine geeigneten Reagenzien der Struktur A-X-B bekannt, deren Gruppen A und B eine ausreichende Selektivität aufweisen und deren Gruppen A direkt mit Hydroxygruppen eines Hydrogels reagieren können.

Die erfindungsgemäß verwendeten Biomoleküle weisen eine Aminogruppe, bevorzugt eine primäre oder sekundäre Aminogruppe, oder eine Thiogruppe auf. Beispiele für geeignete Biomoleküle sind Proteine, endständig aminofunktionalisierte Nucleotide oder Polynucleotide. Typischerweise weisen die Biomoleküle die Funktion von Rezeptoren auf. Beispiele sind Antikörper (z. B. IgGs) oder Antigene für bestimmte Antikörper, aber auch Substrate für Enzyme. Besonders eignen sich die erfindungsgemäßen Gegenstände zur Detektion von Rezeptor-Ligand-Wechselwirkungen über Verfahren, die auf Affinitätswechselwirkung basieren.

Die Bedingungen, bei der die kovalente Anbindung des Biomoleküls mit Amino- oder Thiogruppen an den Gegenstand erfolgt, variieren in Abhängigkeit von dem gewählten System. Typischerweise erfolgt die Anbindung bei Raumtemperatur und in wässrigen Lösungen. Typische Reaktionsdauern betragen 10 Minuten bis 2 Stunden. Die organischen Moleküle werden in einer Konzentration von 10 µg·ml⁻¹ bis zu 500 µg-ml⁻¹ verwendet.

Die Erfindung wird durch die folgenden Beispiele näher erläutert.

### BEISPIELE

### ALLGEMEINES:

Wasserfreie Lösungsmittel (Fa. SDS) auf Molekularsieben (3-4 Ä) wurden so verwendet wie erhalten. Säulenchromatographie (Fa. CC): Silicagel 60 (0,040 - 0,063 mm) der Fa. Merck oder Silicagel der Fa. SDS. Analytische und Dünnschichtchromatographie (DC): Silicagelplatten der Fa. Merck; Erfassung durch UV (254 nm), I₂, 5% H₂SO₄ oder [MoO₄(NH₄)₂ (2,5 g), (NH₄)₂Ce(NO₃)₆ (1,2 g), H₂SO₄ (100 ml, 3,6 M)]. Schmelzpunkt (Smp): Büchi 510. ¹H-NMR und ¹³C-NMR Spektren: AM-250 Bruker; chemische Verschiebungen in ppm bezogen auf protoniertes Lösungsmittel als internen Referenzwert (¹H: CHCl₃ in CDCl₃, 7,27 ppm; CHD₂SOCD₃ in CD₃SOCD₃, 2,49 ppm. ¹³C: ¹³CDCl₃ in CDCl₃, 76,9 ppm, ¹³CD₃SOCD₃ in CD₃SOCD₃, 39,6 ppm); Kopplungskonstanten J in Hz. Die massenspektrometrischen Untersuchungen wurde im Service de Spectrometrie de masse de l'ENS durchgeführt. Die Mikroanalysen wurden durchgeführt vom Service de Microanalyses de l'Université Pierre et Marie Curie, Paris.

### BEISPIEL 1: Herstellung von Succinimidyldiazoessigester über Glyoxylsäure p-Toluolsulfonylhydrazon

### Glyoxylsäure p-Toluolsulfonylhydrazon 1

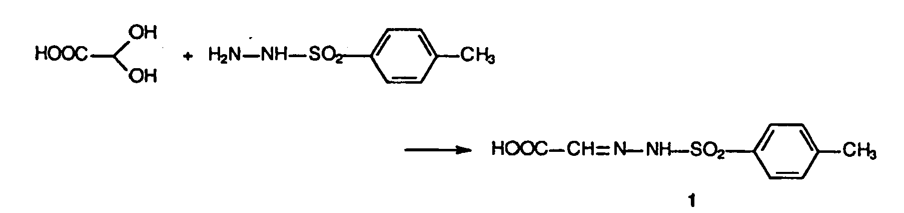

Eine Lösung von 80% Glyoxylsäure (15,1 g, 0,164 mol) in Wasser (162 ml) wird in einen Rundkolben gegeben und im Wasserbad auf 60 °C erwärmt. Dann wird eine warme (60 °C) Lösung von *p*-Toluolsulfonylhydrazid (30,37 g, 0,163 mol) in wäßriger 2,5 M Salzsäure (82 ml) hinzugefügt. Die entstandene Mischung wird im Wasserbad (60 °C) unter ständigem Rühren erhitzt. Es bilden sich sofort Öltropfen. Das anfangs als Öl abgeschiedene Hydrazon verfestigt sich nach ca. 10 Minuten. Die Reaktionsmischung wird langsam auf Raumtemperatur abgekühlt und dann bei 4 °C 6 Stunden stehengelassen. Das rohe *p*-Toluolsulfonylhydrazon wird filtriert, mit kaltem Wasser gewaschen und im Hochvakuum einen Tag getrocknet. Die Verbindung ist dann auskristallisiert. Sie wird in kochendem Ethylacetat (130 ml) aufgelöst und welches dann mit Tetrachlorkohlenstoff (260 ml) verdünnt wird. Nach einer Nacht bei 4 °C wird aus der Suspension das reine *p*-Toluolsulfonylhydrazon 1 als weißer Feststoff herausgefiltert.

Ausbeute: 33,2 g, 84%
Smp: 150 °C.
¹H-NMR (DMSO, 250 MHz): δ (ppm): 2,21 (s, 3H, CH₃); 7,02 (s, 1H, NH); 7,27, 7,55 (2d, 4H, *J* 8,2 Hz, H-ar); 12,12 (s, 1H, =CH).

### Succinimidyldiazoessigester 2

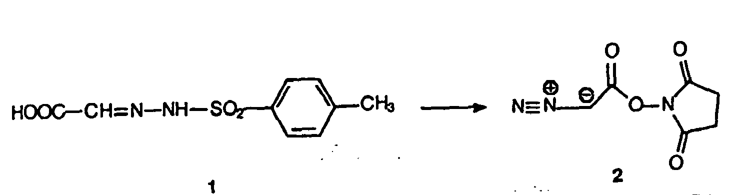

Eine Lösung von Dicyclohexylcarbodiimid (1,7 g, 8,264 mmol) in Dioxan (16 ml) wird tropfenweise zu einer Lösung von *N*-hydroxysuccinimid (0,951 g, 8,264 mmol) und Gtyoxylsäuretosylhydrazon 1 (2 g, 8,264 mmol) in kaltem (0 °C) Dioxan (83 ml) hinzugefügt. Die Mischung wird auf Raumtemperatur gebracht und bei Raumtemperatur 4h gerührt. Die entstandene Suspension wird filtriert. Das Filtrat wird bei Unterdruck konzentriert und das Rohprodukt anschließend chromatographisch auf Silicagel mit Dichlormethan als Elutionsmittel gereinigt. Durch anschließende Kristallisation der Verbindung aus CH₂Cl₂/Hexan, Auflösung in einer geringen Menge von kochendem CH₂Cl₂ und Hinzufügen von Hexan gewinnt man Succinimidyldiazoessigester 2 als weißen Feststoff.

Ausbeute: 0,759 g, 39%
Smp: 118 °C.
¹H-NMR (CDCl₃, 250 MHz): δ (ppm): 2,85 (s, 4H, 2 CH₂ Succinimid); 5,12 (s breit, 1H, CH-). ¹³C-NMR (CDCl₃, 62,90 MHz): δ (ppm): 25,40 (CH₂-Succinimid); 45,03 (CH₂-Succinimid); 162,00 (CO-Diazoacetyl); 169,30 (CO-Succinimid).

### BEISPIEL 2: Herstellung von Succinimidyldiazoessigester über Glyoxylsäurechlorid-p-toluoisulfonylhydrazon

### Glyoxylsäurechlorid-p-toluolsulfonylhydrazon 3

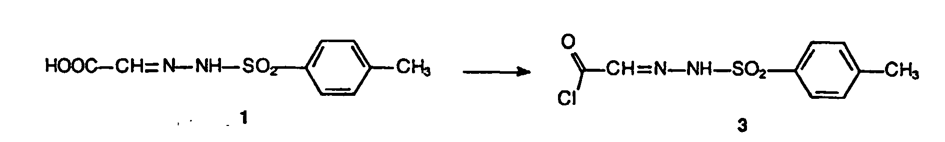

Thionylchlorid (7,2 ml) wird zu einer Suspension von Glyoxylsäure-*p-*Toluolsulfonylhydrazon 1 (12 g, 49,54 mmol) in trockenem Benzol (60 ml) hinzugefügt. Die Mischung wird in Stickstoffatmosphäre 5 Minuten gerührt und dann unter Rückfluß erhitzt, bis die starke Gasentwicklung (HCl, SO₂) beendet ist und sich der größte Teil des suspendierten Feststoffs aufgelöst hat. Nach 60 bis 90 Minuten färbt sich die anfangs weiße Suspension gelb. Die Mischung wird dann sofort abgekühlt und über Celite^{®} filtriert. Nach Konzentration des Filtrats bei Unterdruck wird der verbleibende Feststoff in einer geringen Menge kochendem wasserfreien Benzol aufgelöst. Petrolether (30 - 60 °C) wird zur heißen Lösung hinzugefügt. Die Kristallisation setzt mit der Abkühlung ein. Nach 1 Stunde wird das Hydrazon 3 durch Filtrieren gewonnen.

Ausbeute: 9,8 g, 76%
Smp: 100 - 110 °C.
¹H-NMR (CDCl₃, 250 MHz): δ (ppm): 2,30 (s, 3H, CH₃); 7,20 (s, 1H, NH); 7,39, 7,67 (2d, 4H, *J* 8,25 Hz, H-ar), 12,36 (s, 1H, =CH).

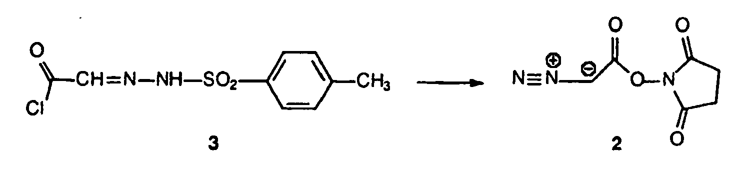

### Succinimidyldiazoessigester 2

Eine Lösung von Glyoxylsäurechlorid-*p*-toluoisulfonylhydrazon **3** (9,8 g, 37,63 mmol) in wasserfreiem Dichlormethan (95 ml) wird im Laufe von 45 Minuten zu einer gerührten und auf 0 °C gehaltenen Suspension von *N*-Hydroxysuccinimid (6 g, 52,17 mmol) und trockenem Na₂CO₃ (7,54 g, 71,16 mmol) in trockenem Dichlormethan (72 ml) hinzugefügt. Nach dem Hinzufügen wird die entstandene Suspension auf Raumtemperatur gebracht und bei Raumtemperatur 3 Stunden gerührt. Die Reaktionsmischung wird erst durch einen Sandfilter und dann durch Celite^{®} filtriert. Das Filtrat wird bei Unterdruck konzentriert. Durch anschließende Rekristallisation der Rohverbindung aus Dichlormethan/Hexan, Auflösung in einer geringen Menge kochendem Dichlormethan und Hinzufügen von Hexan gewinnt man Succinimidyldiazoessigester 2 (Ausbeute: 3,44 g, 50%).

### BEISPIEL 3: Reaktivität von Succinimidyldiazoessigester

### 1. Sequentielle Reaktivität auf primäre Alkohole sowie primäre Amine

### a) Reaktion mit Alkohol

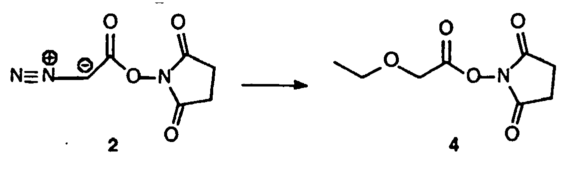

Absoluter Ethylalkohol (5 ml) wird zu einer Lösung von Succinimidyldiazoessigester **2** (400 mg, 2,186 mmol) in wasserfreiem Dichlormethan (4 ml) bei Raumtemperatur hinzugefügt. Die Lösung wird mit Stickstoff durchgespült, danach wird Bortrifluoridetherat (83 µl) langsam hinzugefügt. Die Lösung wird bei Raumtemperatur 3,5 Stunden gerührt. Nach Verdampfen des Lösungsmittels bei Unterdruck wird der Rückstand mit Dichlormethan extrahiert. Die organische Phase wird mit Wasser und einer 1 %-igen Natriumhydroxidlösung gewaschen, über Magnesiumsulfat getrocknet und anschließend konzentriert. Der Ether-NHS-Ester **4** wird im Hochvakuum eine Nacht getrocknet. Eine Reaktion zwischen Alkohol und der difunktionellen organischen Verbindung wird in keinem der Beispiele beobachtet.

Ausbeute: 307 mg, 70%
¹H-NMR (CDCl₃, 250 MHz): δ (ppm): 4,42 (s, 2H, CO-CH₂-*O*); 3,67 (q, 2H, J 7 Hz,
*O*-CH₂-CH₃); 2,87 (s, 4H, CH₂-Succinimid); 1,27 (t, 3H, *O*-CH₂-CH₃).
¹³C-NMR (CDCl₃, 62,90 MHz): δ (ppm): 14,91 (CH₃), 25,60 (2 CH₂-Succinimid), 65,87 (O-CH₂-CH₃), 67,87 (CO-CH₂-*O*). 166,06 (COO), 168,79 (2 CO-Succinimid).
MS (CI/NH₃) m/z 219 (M+18). HRMS (Cl,CH₄) (M+1): m/z: berechnet für C₈H₁₂NO₅: 202,0715. Festgestellt 202,0707.

### b) Reaktion mit Aminen

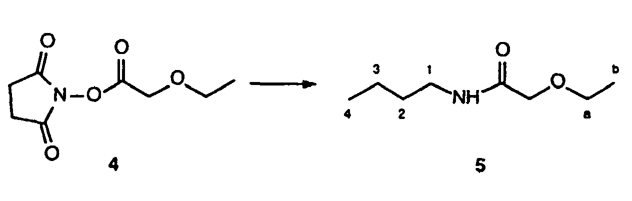

*n*-Butylamin (42 µl, 0,428 mmol) wird zu einer Lösung des Alkoholkonjugats **4** (43 mg, 0,214 mmol) in wasserfreiem Tetrahydrofuran (1,5 ml) unter Stickstoffatmosphäre hinzugefügt. Die Mischung wird bei Raumtemperatur 3 Stunden gerührt. Nach Verdampfen des Lösungsmittels bei Unterdruck wird der Rückstand mit Dichlormethan extrahiert. Die organische Phase wird zweimal mit Wasser gewaschen und über Magnesiumsulfat getrocknet. Die entstandene Verbindung 5 wird im Hochvakuum 2 Minuten getrocknet. Wegen seiner Flüchtigkeit läßt sich nach dem Isolieren keine genaue Ausbeute angeben.

¹H-NMR (CDCl₃, 250 MHz): δ (ppm): 0,87 (t, 3H, *J*_{3,4} 7,24 Hz, CH₃⁴); 1,19 (t, 3H, *J*_{a,b} 7,03 Hz, CH₃^{b}); 1,53-1,12 (m, 4H, CH₂² and CH₂³); 3,23 (dd, 2H, *J*_{1,2a} 6,76 Hz, *J*_{1,2b} 13,23 Hz, CH₂¹); 3,49 (q, 2H, *J*_{a,b} 7,01 Hz, CH₂^{a}); 3,85 (s, 2H, CO-CH₂-*O*); 6,5 (s, 1 H, NH).
¹³C-NMR (CDCl₃, 62,90 MHz): δ (ppm): 13,74 (CH₃⁴), 15,06 (CH₃^{b}), 20,09 (CH₂³), 31,70 (CH₂²), 38,56 (CH₂¹-NH), 67,11 (*O*-CH₂^{a}), 69,98 (O-CH₂-CO), 170,00 (CONH).
MS (Cl/NH₃) m/z 160 (M+1), 177 (M+18). HRMS (Cl,CH₄) (M+1): m/z: berechnet für C₈H₁₈NO₂: 160,1338. Festgestellt 160,1348.

### 2. Sequentielle Reaktivität auf sekundäre Alkohole sowie sekundäre Amine

### a) Reaktion mit Alkohol

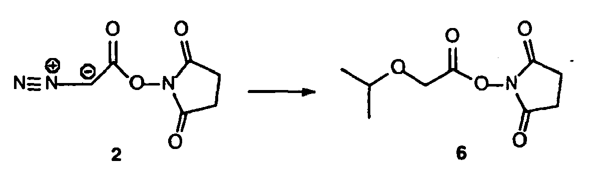

Absoluter Isopropylalkohol (1,5 ml) und Bortrifluoridetherat (50 µl) werden nacheinander unter Stickstoffatmosphäre zu einer Lösung von Succinimidyldiazoessigester **2** (130 mg, 0,71 mmol) in wasserfreiem Dichlormethan (1,5 ml) hinzugefügt. Die Mischung wird bei Raumtemperatur 3 Stunden gerührt und dann unter Rückfluß 1,5 Stunden erwärmt (40 °C). Nach Verdampfen des Lösungsmittels bei Unterdruck wird der Rückstand mit Dichlormethan extrahiert. Die organische Phase wird mit Wasser und einer Natriumhydroxidlösung gewaschen, über Magnesiumsulfat getrocknet und schließlich konzentriert. Die getrocknete Verbindung wird in kochendem Dichlormethan aufgelöst. Nach anschließender Verdünnung mit Hexan gewinnt man das kristalline Alkoholkonjugat 6.

Ausbeute: 107 mg, 70%
Smp: 49,5 - 49,8 °C.
¹H-NMR (CDCl₃, 250 MHz): δ (ppm): 1,23 (d, 6H, J 6,11 Hz, 2 CH₃iso); 2,86 (s, 4H, 2 CH₂-Succinimid); 3,76 (m, 1 H, CH-iso); 4,43 (s, 2H, CO-CH₂-*O*).
¹³C-NMR (CDCl₃, 62,90 MHz): δ (ppm): 21,72 (2 CH₃iso), 25,60 (2 CH₂-Succinimid), 63,60 (CH-iso), 73,57 (CO-CH₂-*O*), 166,48 (COO), 168,83 (2 CO-Succinimid).
MS (CI/NH₃) m/z_233 (M+18).
Analyse: berechnet C₉H₁₃O₅N: C, 50,23; H, 6,088; N, 6,508. Festgestellt: C, 50,20; H, 6,21; N, 6,53.

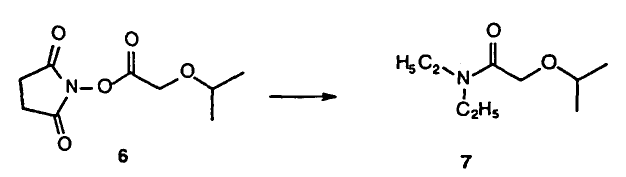

### b) Reaktion mit Diethylamin

Diethylamin (42 µl, 0,404 mmol) wird zu einer Lösung des Alkoholkonjugats 6 (43,4 mg, 0,202 mmol) in wasserfreiem Tetrahydrofuran (1,5 ml) unter Stickstoffatmosphäre hinzugefügt. Die Lösung wird bei Raumtemperatur 3 Stunden gerührt. Nach Verdampfen des Lösungsmittels bei Unterdruck wird der Rückstand mit Dichlormethan extrahiert. Die organische Phase wird mit Wasser gewaschen und über Magnesiumsulfat getrocknet. Das entstandene Konjugat 7 wird im Hochvakuum 2 Minuten getrocknet. Diese flüssige Verbindung läßt sich leicht destillieren; eine genaue Ausbeute kann nicht angegeben werden.

¹H-NMR (CDCl₃, 250 MHz): δ (ppm): 1,06 (t, 3H, *J* 7,13 Hz, CH₃amin); 1,10 (t, 3H, *J* 7,5 Hz, CH₃amin); 1,13 (d, 6H, *J* 6,13 Hz, 2 CH₃iso); 3,28 (q, 2H, CH₂amin); 3,31 (q, 2H, CH₂amin); 3,63 (m, 1H, CH-iso); 4,05 (s, 2H, CO-CH₂-O).
¹³C-NMR (CDCl₃, 62,90 MHz): δ (ppm): 12,85, 14,27 (2 CH₃amin), 21,91 (2 CH₃iso), 40,05, 41,34 (2 CH₂amin), 68,01 (CH-iso), 72,33 (CO-CH₂-O), 168,98 (CON).
MS (Cl/NH₃) m/z 174 (M+1). HRMS (Cl,CH₄) (M+1): m/z: berechnet für C₉H₂₀NO₂: 174,1494. Festgestellt 174,1476.

### c) Reaktion mit Dioctadecylamin

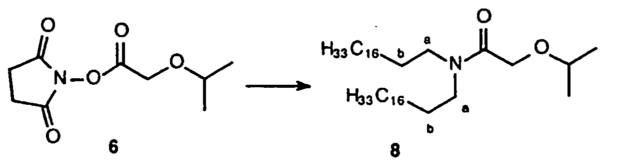

Eine Lösung von Alkoholkonjugat **6** (59,4 mg, 0,277 mmol) und Dioctadecylamin (144 mg, 0,277 mmol) in wasserfreiem Tetrahydrofuran (2 ml) wird unter Stickstoffatmosphäre bei 50 °C 3 Stunden gerührt. Nach Verdampfen des Lösungsmittels bei Unterdruck wird der Rückstand mit Dichlormethan extrahiert. Die organische Phase wird mit Wasser gewaschen. Anschließend wird das Lösungsmittel verdampft. Das entstandene Konjugat **8** wird im Hochvakuum **3** Stunden getrocknet.

Ausbeute: 150 mg, 87%
¹H-NMR (CDCl₃, 250 MHz): δ (ppm): 0,89 (t, 6H, J 6,89 Hz, 2 CH₃amin); 1,20 (d, 6H, *J* 6,11 Hz, 2 CH₃iso); 1,26 (s, 60H, 30 CH₂); 1,53 (m, 4H, 2 CH₂^{b}); 3,26 (m, 4H, 2 CH₂^{a}); 3,69 (m, 1H, CH-iso); 4,11 (s, 2H, CO-CH₂-O).
¹³C-NMR (CDCl₃, 62,90 MHz): δ (ppm): 14,12 (2 CH₃amin), 21,91 (2 CH₃iso), 22,72, 26,95, 27,10, 27,56, 29,00, 29,39, 29,46, 29,62, 29,70, 29,73, 31,96 (32 CH₂), 45,77, 47,20 (2 CH₂^{a}), 67,86 (CH-iso), 72,18 (CO-CH₂-*O*), 169,22 (CO).
MS (Cl/NH₃) m/z 622 (M+1). HRMS (Cl,CH₄) (M+1): m/z: berechnet für C₄₁H₈₄NO₂: 622,6502. Festgestellt 622,6490.

### BEISPIEL 4: Herstellung von 4-Vinylsulfonylbenzoylchlorid

### 1-(2-Chlorethansulfonyl)-4-methylbenzol 9

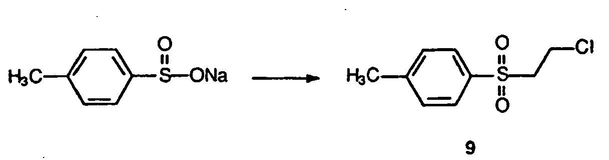

1-Brom-2-chlorethan (11 ml, 0,132 mol) wird zu einer Lösung des Natriumsalzes der Toluol-4-sulfinsäure (19,623 g, 0,11 mol) in trockenem Dimethylformamid (180 ml) hinzugefügt. Die Reaktionsmischung wird bei Raumtemperatur 2 Tage gerührt. Nach Verdampfen des Lösungsmittels bei Unterdruck wird der rohe Rückstand mit Dichlormethan extrahiert. Die organische Phase wird mit Wasser gewaschen, über Magnesiumsulfat getrocknet und konzentriert. Durch anschließende Rekristallisation des Rückstands in kochendem 80%igem Ethanol gewinnt man 1-(2-Chlorethansulfonyl)-4-methylbenzoi 9 als weiße Kristalle.

Ausbeute: 18,58 g, 77%
Smp: 78 °C.
¹H-NMR (CDCl₃, 250 MHz): δ (ppm): 2,48 (s, 6H, CH₃); 3,52 (t, 2H, J 7,5 Hz, CH₂SO₂); 3,74 (t, 2H, CH₂Cl); 7,40, 7,80 (2d, 4H, *J*8,30 Hz, H-ar).

### 4-(2-Chlorethansulfonyl)benzoesäure 10

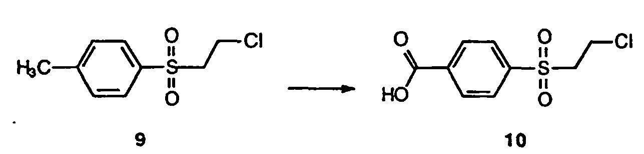

Chromtrioxid (14 g) und konzentrierte Schwefelsäure (9,6 ml) werden nacheinander zu einer Lösung von 1-(2-Chlorethansulfonyl)-4-methylbenzol **9** (7,687 g, 35,15 mmol) in Essigsäure (115 ml) hinzugefügt. Die Mischung wird bei Raumtemperatur 3 Stunden gerührt und dann in Eiswasser gegossen. Aus dem weißen Niederschlag wird durch Filtrieren, Waschen und Rekristallisation 4-(2-Chlorethansulfonyl)benzoesäure **10** gewonnen.

Ausbeute: 7,16 g, 82%
Smp: 220 °C.
¹H-NMR (DMSO, 250 MHz): δ (ppm): 3,97 (t, 2H, *J* 6,5 Hz, CH₂SO₂); 4,12 (t, 2H, CH₂Cl); 8,23, 8,34 (2d, 4H, *J* 8,30 Hz, H-ar), 13,66 (s breit, 1H, OH).

### 4-Vinylsulfonylbenzoesäure 11

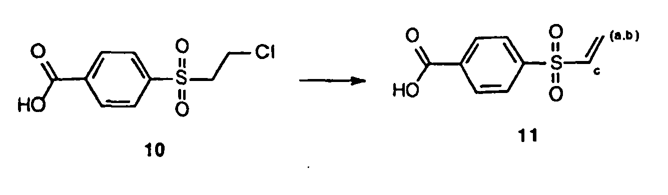

Triethylamin (8,03 ml, 57,6 mmol) wird zu einer Lösung von 4-(2-Chlorethansulfonyl)benzoesäure **10** (7,16 g, 28,8 mmol) in Chloroform (144 ml) hinzugefügt. Die Mischung wird über Nacht bei Raumtemperatur gerührt. Nach Verdampfen des Lösungsmittels bei Unterdruck wird der Rückstand in Wasser aufgelöst und filtriert, um die unlöslichen Teile abzuscheiden. Zu dem Filtrat wird konzentrierte Salzsäure hinzugefügt. Aus dem Niederschlag wird durch Rekristallisation in Wasser, anschließendes Filtrieren und Trocknen im Hochvakuum die 4-Vinylsulfonylbenzoesäure **11** gewonnen.

Ausbeute: 5,10 g, 84%
Smp: 228 °C.
¹H-NMR (DMSO, 250 MHz): δ (ppm): 6,26 (d, 1H, *J*_{a,c} 9,92 Hz, CHa=); 6,38 (d, 1H, *J*_{b,c} 16,48 Hz, CHb=); 7,15 (dd, 1 H, CHc=); 7,96, 8,14 (2d, 4H, *J* 8,50 Hz, H-ar); 13,56 (s, 1 H, OH).
¹³C-NMR (DMSO, 62,90 MHz): δ (ppm): 128,02, 130,69 (4 CH-ar), 130,20, 138,25 (CH=, CH₂=), 135,73, 143,39 (2 Cq-ar), 166,32 (CO).

### 4-Vinylsulfonylbenzoylchlorid 12

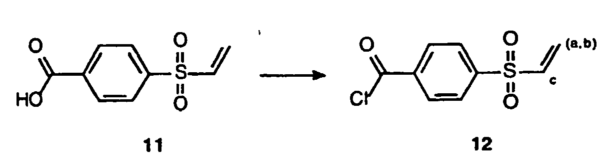

Eine katalytische Menge trockenes Dimethylformamid (38 µl, 0,488 mmol) wird unter Stickstoffatmosphäre zu einer Lösung von 4-Vinylsulfonylbenzoesäure **11** (230 mg, 1,084 mmol) in Thionylchlorid (4,6 ml) hinzugefügt. Die Mischung wird unter Rückfluß 3 Stunden erhitzt (85 °C). Die Lösung wird zur Trockene eingedampft, dann jeweils zweimal mit trockenem Toluol aufgenommen und bei Unterdruck zur Trockene eingedampft und schließlich 2 Stunden im Hochvakuum getrocknet.

¹H-NMR (DMSO, 250 Mhz): δ (ppm): 6,30 (d, 1 H, *J*_{a.c} 9,85 Hz, CHa=); 6,42 (d, 1H, *J*_{b.c} 16,47 Hz, CHb=); 7,2 (dd, 1H, CHc=); 8,00, 8,18 (2d, 4H, J 8,54 Hz, H-ar).

### BEISPIEL 5: Reaktivtät von 4-Vinylsulfonylbenzoylchlorid

### 1. Sequentielle Reaktivität auf primäre Alkohole sowie primäre Amine

### a) Reaktion mit Alkohol:

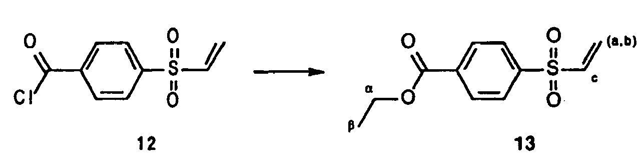

Absoluter Ethylalkohol (76 µl, 1,3 mmol) und *N*,*N*-Diisopropylethylamin (170 µl, 0,976 mmol) werden nacheinander zu einer Lösung von 4-Vinylsulfonylbenzoylchlorid **12** in wasserfreiem Dichlormethan (4 ml) hinzugefügt. Die Mischung wird bei Raumtemperatur über Nacht gerührt. Nach Verdampfen des Lösungsmittels bei Unterdruck wird der Rückstand mit Dichlormethan extrahiert. Die organische Phase wird mit Wasser gewaschen, über Magnesiumsulfat getrocknet und schließlich zur Trockene eingedampft. Nach Reinigen des Rückstands durch Säulenchromatographie auf Silicagel mit Dichlormethan als Elutionsmittel gewinnt man das analytisch reine Alkoholkonjugat **13**.

Ausbeute: 206 mg, 79%
Smp: 39 - 40 °C.
¹H-NMR (DMSO, 250 MHz): δ (ppm): 1,51 (t, 3H, J 7,14 Hz, CH₃^{B}); 4,53 (q, 2H, CH_{2α}); 6,48 (d, 1H, *J*_{a,c} 9,85 Hz, CHa=); 6,59 (d, 1H, *J*_{b.c}16,46 Hz, CHb=); 7,37 (dd, 1 H, CHc=); 8,2, 8,36 (2d, 4H, J 8,43 Hz, H-ar).
¹³C-NMR (DMSO, 62,90 MHz): δ (ppm): 14,51 (CH₃), 61,98 (CH_{2α}), 128,33, 130,73 (4 CH-ar), 130,52 (=CH₂), 134,85 (Cq-ar), 138,40 (=CHc), 143,90 (Cq-ar), 164,96 (COO), MS (Cl/NH₃) m/z 258 (M+18).
Analyse: berechnet C₁₁H₁₂O₄S: C, 54,98; H, 5,033. Festgestellt: C, 55,43; H, 4,96.

### b) Reaktion mit n-Butylamin:

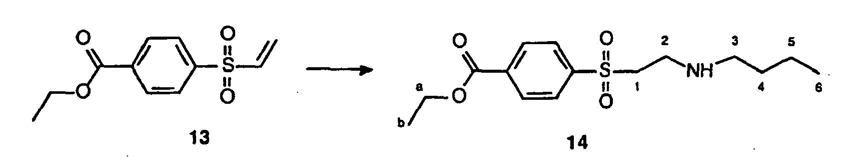

Das Alkoholkonjugat **13** (64,6 mg, 0,269 mmol) und *n*-Butylamin (133 µl, 1,345 mmol) werden in absolutem Ethanol (2 ml) 12 Stunden bei Raumtemperatur gerührt. Durch Verdampfen des Lösungsmittels und des überschüssigen *n-*Butylamin gewinnt man das Alkoholaminkonjugat **14,** das für die Mikroanalyse durch Säulenchromatographie auf Silicagel mit Dichlormethan/Methanol: 20/1 als Elutionsmittel gereinigt wird.

Ausbeute: 75,8 mg, 90%
¹H-NMR (CDCl₃, 250 MHz): δ (ppm): 0,9 (t, 3H, *J*_{6,5} 7,1 Hz, CH₃⁶); 1,22 - 1,49 (m, 4H, CH₂⁴+CH₂⁵); 1,42 (t, 3H, *J*_{a,b} 7,14 Hz, CH₃^{b}); 2,56 (t, 2H, *J*_{3,4} 7,07 Hz, CH₂³); 3,03 (t, 2H, *J* _{1.2} 6,42 Hz, CH₂²); 3,33 (t, 2H, CH₂¹); 4,435 (q, 2H, *O*-CH₂^{a}); 7,21 (m, 1H, NH); 8,00, 8,23 (2d, 4H, *J*8,45 Hz, H-ar).
¹³C-NMR (CDCl₃, 62,90 MHz): δ (ppm): 13,918, 14,259 (2 CH₃), 20,326 (CH₂⁵), 31,982 (CH₂⁴), 43,064 (CH₂¹), 49,270 (CH₂²), 61,850 (*O*-CH₂^{a}), 128,078 (2 CH-ar), 130,426 (2 CH-ar), 135,335 (Cq-ar), 143,138 (Cq-ar), 164,964 (COO).
MS (Cl/NH₃) m/z 314 (M+1).
Analyse: berechnet C₁₅H₂₃O₄NS: C, 57,48; H, 7,396; N, 4,469. Festgestellt: C, 57,53; H, 7,41; N 4,31.

### c) Reaktion mit tert-Butylamin:

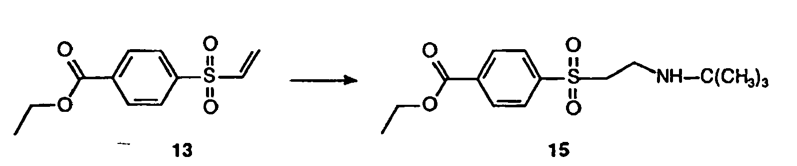

Das Alkoholkonjugat **13** (54,7 mg, 0,228 mmol) und *tert*-Butylamin (120 µl, 1,139 mmol) werden in absolutem Ethanol (4 ml) 12 Stunden bei Raumtemperatur gerührt. Durch Verdampfen des Lösungsmittels und des überschüssigen *tert-*Butylamin gewinnt man das Konjugat 15, das für die Mikroanalyse durch Säulenchromatographie auf Silicagel mit Dichlormethan/Methanol: 20/1 als Elutionsmittel gereinigt wird.

Ausbeute: 64,1 mg, 90%
¹H-NMR (CDCl₃, 250 MHz): δ (ppm): 1,07 (s, 9H, (CH₃)₃); 1,43 (t, 3H, *J* 7,13 Hz, CH₂-CH₃); 2,98 (t, 2H, J 6,42 Hz,CH₂-SO₂); 3,31 (t, 2H, CH₂-NH); 4,43 (q, 2H, *O*-CH₂); 7,22 (m, 1H, NH); 8,00, 8,23 (2d, 4H, J 8,39 Hz, H-ar).
¹³C-NMR (CDCl₃, 62,90 MHz): δ (ppm): 14,25 (CH₂-CH₃), 28,81 ((CH₃)₃), 36,38 (CH₂-SO₂), 50,75 (Cq-aliphatisch), 57,27 (CH₂-NH), 61,85 (*O*-CH₂), 128,09 (2 CH-ar), 130,38 (2 CH-ar), 135,30 (Cq-ar), 143,25 (Cq-ar), 164,98 (COO).
MS (CI/NH₃) m/z 314 (M+1).
Analyse: berechnet C₁₅H₂₃O₄NS: C, 57,48; H, 7,396; N, 4,469. Festgestellt: C, 57,55; H, 7,44; N, 4,39.

### 2. Sequentielle Reaktivität auf sekundäre Alkohole sowie sekundäre Amine

### a) Reaktion mit Alkohol:

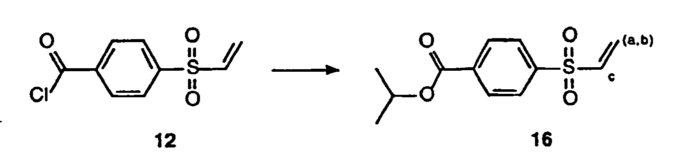

Absoluter Isopropylalkohol (202 µl, 2,638 mmol) und *N*,*N*-Diisopropylethylamin (206,8 µl, 1,187 mmol) werden nacheinander zu einer Lösung von 4-Vinylsulfonylbenzoylchlorid **12** (304,3 mg, 1,319 mmol) in wasserfreiem Dichlormethan (3 ml) hinzugefügt. Die Mischung wird bei Raumtemperatur über Nacht gerührt. Um die Reaktion zu Ende zu führen, wird der Lösung eine katalytische Menge Dimethylaminopyridin zugesetzt. Nach einer Stunde wird das Lösungsmittel bei Unterdruck verdampft und dann der Rückstand mit Dichlormethan extrahiert. Die organische Phase wird mit Wasser gewaschen, über Magnesiumsulfat getrocknet und schließlich zur Trockene eingedampft. Nach Reinigen des Rückstands durch Säulenchromatographie auf Silicagel mit Dichlormethan als Elutionsmittel gewinnt man das reine Alkoholkonjugat **16**.

Ausbeute: 247 mg, 74%
Smp: 57 - 58 °C.
¹H-NMR (CDCl₃, 250 MHz): δ (ppm): 1,38, 1,41 (2s, 6H, 2 CH₃iso); 5,28 (m, 1H, CH-iso); 6,11 (d, 1H, *J*_{a,c} 9,44 Hz, CHa=); 6,51 (d, 1H, *J*_{b,c} 16,5 Hz, CHb=); 6,68 (dd, 1H, CHc=); 7,97, 8,20 (2d, 4H, J 8,70 Hz, H-ar).
¹³C-NMR (COCl₃, 62,90 MHz): δ (ppm): 21,67 (2 CH₃iso), 69,54 (CH-iso), 127,91 (2 CH-ar), 128,84 (CH₂=), 130,43 (2 CH-ar), 135,60 (Cq-ar), 136,05 (CHc=), 143,30 (Cq-ar), 164,47 (COO).
MS (Cl/NH₃) m/z 272 (M+18).

Analyse: berechnet C₁₂H₁₄O₄S: C, 56,67; H, 5,549. Festgestellt: C, 56,65; H, 5,55.

### b) Reaktion mit Amin:

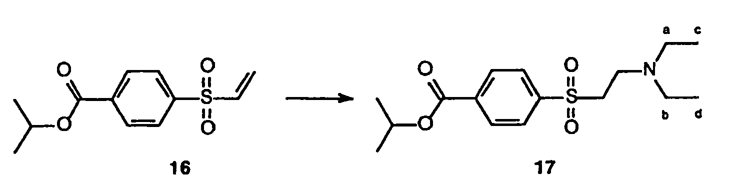

Das Alkoholkonjugat **16** (72,6 mg., 0,286 mmol) und Diethylamin (148 µl, 1,431 mmol) werden in absolutem Ethanol (2,5 ml) 12 Stunden bei Raumtemperatur gerührt. Durch Verdampfen des Lösungsmittels und des überschüssigen Diethylamin gewinnt man das Alkohol-Amin-Konjugat **17**, das für die Mikroanalyse durch Säulenchromatographie auf Silicagel mit Dichlormethan/Methanol 30/1 als Elutionsmittel gereinigt wird.

Ausbeute: 84 mg, 90%)
¹H-NMR (CDCl₃, 250 MHz): δ (ppm): 0,92 (t, 6H, *J*_{a,c} = *J*_{b,d} 7,12 Hz, CH₃ b); 1,40, 1,42 (2s, 6H, CH₃iso); 2,42 (q, 4H, CH₂ a); 2,90 (m, 2H, CH₂SO₂); 3,28 (m, 2H, CH₂-N); 5,29 (m, 1 H, CH-iso); 7,99, 8,22 (2d, 4H, J 8,64 Hz, H-ar).
¹³C-NMR (CDCl₃, 62,90 MHz): δ (ppm): 11,73 (2 CH₃amin), 21,68 (2 CH₃iso), 45,86 (CH₂-SO₂), 46,75 (2 CH₂amin), 53,35 (CH₂N), 69,56 (CH-iso), 128,01 (2 CH-ar), 130,26 (2 CH-ar), 135,57 (Cq-ar), 143,34 (Cq-ar), 164,51 (COO).
MS (Cl/NH₃) m/z 328 (M+1).
Analyse: berechnet C₁₆H₂₅O₄NS: C, 58,69; H, 7,695; N, 4,277. Festgestellt: C, 58,69; H, 7,70; N 4,28.

### 3. Sequentielle Reaktivität auf primäre Alkohole sowie primäre Thiole

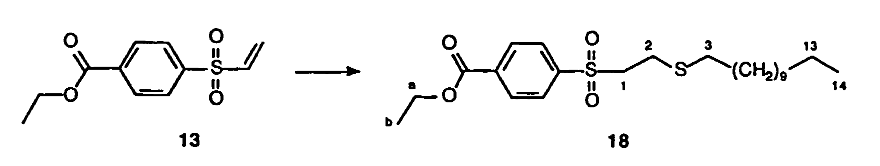

Das erhaltene Alkoholkonjugat **13** (104 mg, 0,434 mmol) und 1-Dodecanthiol (104 µl, 0,434 mmol) werden bei Raumtemperatur in absolutes Ethanol gegossen (4 ml), und Triethylamin (18 µl, 0,130 mmol) wird hinzugefügt. Die Mischung wird bei Raumtemperatur 2 Stunden gerührt. Das Alkohol-Thiol-Konjugat kristallisiert bei seiner Bildung im Ethanol aus. Aus der kristallinen Verbindung gewinnt man durch Filtrieren, Waschen mit wenig Ethanol und anschließendes Trocknen im Hochvakuum das Alkohol-Thiol-Konjugat **18**.

Ausbeute: 165 mg, 86%
Smp: 78 - 79 °C.
¹H-NMR (CDCl₃, 250 MHz): δ (ppm): 0,88 (t, 3H, *J*_{13,14} 7 Hz, CH₃¹⁴); 1,26 (s, 18H, (CH₂)₉); 1,42 (t, 3H, *J*_{a,b} 7,09 Hz, CH₃^{b}); 1,5 (m, 2H, CH₂); 2,48 (t, 2H, *J* 7,24 Hz, CH₂³-S); 2,8 (m, 2H, CH₂²-S); 3,34 (m, 2H, CH₂¹-SO₂); 4,44 (q, 2H, CH₂^{a}); 7,99 (d, 2H, *J* 8,3 Hz, H-ar); 8,24 (d, 2H, H-ar).
¹³C-NMR (CDCl₃, 62,90 MHz): δ (ppm): 14,12, 14,26 (2 CH₃), 22,70, 24,26, 28,75, 29,15, 29,34, 29,49, 29,57, 29,63 (10 CH₂), 31,92, 32,34 (2 CH₂-S), 56,41 (CH₂¹-SO₂), 61,90 (CH₂^{a}), 128,22 (2 CH-ar), 130,52 (2 CH-ar), 135,53 (Cq-ar), 142,50 (Cq-ar), 164,91 (COO).
MS (Cl/NH₃) m/z 460 (M+18).
Analyse: berechnet C₂₃H₃₈O₄S₂: C, 62,40; H, 8,652. Festgestellt: C, 62,36; H, 8,61.

### BEISPIEL 6: Herstellen eines SPR-Sensors

Eine mit einem Hydrogel funktionalisierte Goldoberfläche eines SPR-Biosensors wird in einer Lösung von 2-Diazoessigsäure-N-hydroxysuccinimidester in trockenem Dichlormethan (5 Gew.-%) unter Inertgasatmosphäre 3 h inkubiert. Anschließend wird nacheinander jeweils einmal mit trockenem Dichlormethan, Isopropanol und Reinstwasser gespült. Nach dem Trocknen im Stickstoffstrom ist die erfindungsgemäße Oberfläche einsatzbereit.

### BEISPIEL 7: Anbinden von Succinimidyldiazoessigester an einen funktionalisierten SPR-Sensor und anschließende kovalente Bindung eines Rezeptors

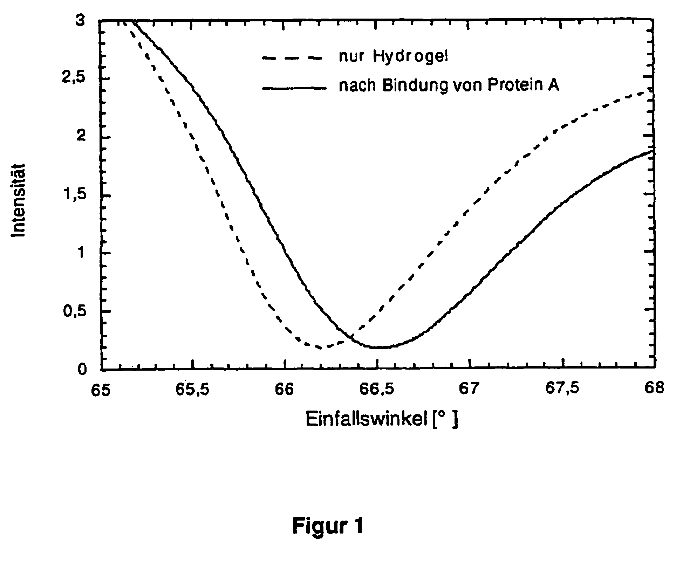

Ein mit Hydrogel funktionalisierter SPR-Sensor wird unter Inertgas bei Raumtemperatur für drei Stunden in eine Lösung von Succinimidyldiazoessigester in trockenem Dichlormethan (5 Gew.-%) getaucht. Nach Spülen mit Dichlormethan, Isopropanol und Wasser wird der Sensor bei Raumtemperatur mit einer wässrigen Lösung von Protein A in Wasser (150 µg•ml⁻¹) inkubiert. Anschließend wird mit viel Wasser gespült. Der Erfolg der Bindung wird durch Oberflächenplasmonenresonanz überprüft. Figur 1 zeigt ein Vergleich der Oberflächenplasmonenresonanzsignale eines Hydrogels vor und nach Bindung von Protein A über Succinimidyldiazoessigester.

## Patentansprüche

1. Gegenstand mit einer ungeladenen, funktionalisierten Oberfläche umfassend ein Hydrogel, das Hydroxygruppen aufweist, an das einen oder mehrere Reste A sowie einen oder mehrere Reste B aufweisende organische Moleküle über die Reste A gebunden sind, wobei die Reste B mit Aminogruppen oder Thiogruppen reagieren können und wobei die Hydroxygruppen selektiv mit dem Rest A bzw. den Resten A reagiert haben.

2. Gegenstand nach Anspruch 1, wobei die Reste A aus Säurechloridgruppen und Diazogruppen ausgewählt sind.

3. Gegenstand nach Anspruch 1 oder 2, wobei die Reste B aus Vinylsulfongruppen, N-Hydroxysuccinimidestergruppen und Maleimidgruppen ausgewählt sind.

4. Gegenstand nach einem der Ansprüche 1 bis 3, wobei die Reste A und B durch eine Einfachbindung oder durch eine verzweigte oder unverzweigte Kohlenwasserstoffkette X verbunden sind und wobei die Kohlenwasserstoffkette X eine Kettenlänge von bis zu 15 Kohlenstoffatomen besitzt und bis zu zweimal jeweils durch eine Phenylengruppe oder eine heteroatomenthaltende Gruppe unterbrochen sein kann.

5. Gegenstand nach einem der Ansprüche 1 bis 3, wobei die organischen Moleküle, welche die Reste A und B aufweisen, Polymere oder Oligomere sind.

6. Verfahren zur Herstellung eines Gegenstandes mit einer ungeladenen, funktionalisierten Oberfläche, umfassend die Schritte:
(a) Bereitstellen eines Gegenstandes mit einer nicht funktionalisierten Hydroge loberfläche, wobei das Hydrogel Hydroxygruppen aufweist;
(b) Kovalentes Anbinden an das Hydrogel von organischen Molekülen, die einen oder mehrere Rest A, die mit Hydroxygruppen reagieren können, und einen oder mehrere Reste B, die mit Aminogruppen oder Thiogruppen rea gieren können, aufweisen,
wobei die organischen Moleküle selektiv über den Rest A bzw. die Reste A direkt mit den Hydroxygruppen des Hydrogels reagieren.

7. Verwendung eines Gegenstandes nach einem der Ansprüche 1 bis 5 zum Umsetzen mit einem Biomolekül mit mindestens einer Aminogruppe oder Thiogruppe.

## Claims

1. Article having an uncharged, functionalized surface which comprises a hydrogel which exhibits hydroxyl groups to which organic molecules possessing one or more radicals A and one or more radicals B are bound by way of the radicals A, wherein the radicals B can react with amino groups or thio groups, and wherein the hydroxyl groups have reacted selectively with the radical or radicals A.

2. Article according to Claim 1, with the radicals A being selected from acid chloride groups and diazo groups.

3. Article according to Claim 1 or 2, with the radicals B being selected from vinyl sulfone groups, N-hydroxysuccinimide ester groups and maleimide groups.

4. Article according to one of Claims 1 to 3, with the radicals A and B being linked by means of a single bond or by means of a branched or unbranched hydrocarbon chain X and with the hydrocarbon chain X having a chain length of up to 15 carbon atoms and being able to be interrupted up to two times by in each case a phenylene group or a heteroatom-containing group.

5. Article according to one of Claims 1 to 3, with the organic molecules possessing the radicals A and B being polymers or oligomers.

6. Process for producing an article having an uncharged, functionalized surface, which process comprises the steps of:
(a) providing an article having an unfunctionalized hydrogel surface, with the hydrogel exhibiting hydroxyl groups;
(b) covalently binding organic molecules which possess one or more radicals A, which can react with hydroxyl groups, and one or more radicals B, which can react with amino groups or thio groups, to the hydrogel,
with the organic molecules reacting selectively directly with the hydroxyl groups of the hydrogel by way of the radical or radicals A.

7. Use of an article according to one of Claims 1 to 5 for reacting with a biomolecule possessing at least one amino group or thio group.

## Revendications

1. Article à surface non chargée et fonctionnalisée, comprenant un hydrogel présentant des groupements hydroxy, auquel sont liées par des radicaux A des molécules organiques comprenant un ou plusieurs radicaux A et un ou plusieurs radicaux B, lesdits radicaux B pouvant réagir avec des groupements amino ou des groupements thio et lesdits groupements hydroxy ayant réagi de manière sélective avec le ou les radicaux A.

2. Article selon la revendication 1, dans lequel les radicaux A sont choisis parmi les groupements chlorure acide et les groupements diazo.

3. Article selon la revendication 1 ou la revendication 2, dans lequel les radicaux B sont choisis parmi les groupements vinylsulfone, les groupements ester N-hydroxysuccinimide et les groupements maléimide.

4. Article selon l'une quelconque des revendications 1 à 3, dans lequel les radicaux A et B sont reliés via une liaison simple ou via une chaîne hydrocarbonée X linéaire ou ramifiée et dans lequel la chaîne hydrocarbonée X présente une longueur de chaîne inférieure ou égale à 15 atomes de carbone et peut être interrompue jusqu'à deux fois par un groupement phénylène ou un groupement contenant un hétéroatome.

5. Article selon l'une quelconque des revendications 1 à 3, dans lequel les molécules organiques, comprenant les radicaux A et B, sont des polymères ou des oligomères.

6. Procédé de fabrication d'un article présentant une surface non chargée et fonctionnalisée, comprenant les étapes :
(a) utilisation d'un objet présentant une surface hydrogel non fonctionnalisée, l'hydrogel comportant des groupements hydroxy ;
(b) liaison covalente à l'hydrogel de molécules organiques comportant un ou plusieurs radicaux A, capables de réagir avec des groupements hydroxy, et un ou plusieurs radicaux B, capables de réagir avec des groupements amino ou des groupements thio,
les molécules organiques réagissant directement de manière sélective par le ou les radicaux A avec les groupements hydroxy de l'hydrogel.

7. Utilisation d'un article selon l'une quelconque des revendications 1 à 5 pour une réaction avec une biomolécule comprenant au moins un groupement amino ou un groupement thio.
